# EUROPEAN PATENT APPLICATION

(11) **EP 3 513 774 A1**
(43) Date of publication of application: **24.07.2019**
(21) Application number: 19161951.9
(22) Date of filing: 29.12.2016
(51) Int. Cl.: A61F 13/20, A61D 7/00, A61M 31/00

(54) **MORPHO-ANATOMIC FLEXIBLE WEB**

(30) Priority: 30.12.2015 RO 201501051
(62) Divisional of application: 16834181.6
(71) Applicant: V-VEIL-UP-PHARMA LIMITED, 1061 Nicosia (CY)
(72) Inventor: CHAFFRINGEON, Bernard, CH-1052 Lausanne (CH)
(74) Representative: Boncea, Oana-Laura

(57) **Abstract**

The present invention relates to a system for delivering, distributing and retaining a liquid, a gel or a cream substance into a vaginal or an anal cavity of a human or animal, comprising an applicator (30) and a flexible web (1) wherein a first portion (1') of the web (1) is folded over a remaining second portion (1") of the web (1) along a folding line (L) and the first (1') and second (1") portions of the web (1) are maintained in a folded position by a plurality of welding regions (23), thereby defining a retaining pouch (2) having lateral edges corresponding to said welding regions (23) and a top opening and a bottom opening (O) for receiving the applicator (30), wherein the top is the side of the pouch closest to the folding line and the bottom is the side of the pouch furthest away from the folding line (L) and a zone (Z) which is delimited by the folding line (L) and by said top opening of the pouch and on the lateral sides by two further openings (O1, O2), situated at the extremities of the folding line (L), wherein the top opening is delimited by two extremities of the welding regions (23) and is configured to receive said applicator (30) and to keep said applicator (30) in a releasably fixed position when its end comprising the at least one orifice is in the zone (Z), in order to deliver the substance into the cavity.

## Description

The present invention relates to the field of devices for distributing and retaining substances inside the bodily cavities, in particular a device and a flexible web for delivering, distributing and retaining a liquid, a gel or a cream substance into a vaginal or an anal cavity of a human or animal.

Document WO2009/112527 discloses a flexible retention web designed to be introduced into a vaginal cavity, made of non-expandable or minimally expandable atraumatic material, the dimensions of which are designed in such a way as to be able to retain and/or slow down bodily discharges of small quantity. The web may be introduced into the desired position by means of a pushing tool. The document discloses the use of this web for absorbing secretions from the vagina when the users produce small volumes of bodily secretions. It does not disclose the use of the web for distributing substances inside the bodily cavities. Moreover, should the web as disclosed by this document be used for distributing substances inside bodily cavities, such substances would not remain inside said cavities for a sufficient amount of time or in a sufficient or controllable amount for the substances to be able to fulfill their function. (Even should the substance be somehow placed inside the pouch defined when one of the flaps passes through the opening (50) of the other flap, it would easily get or flow out through said opening and/or through the lateral openings, and exit the bodily cavity, especially when the user is in a horizontal position).

Substances for intra-vaginal or intra-rectal administration are known, for example having therapeutic effect. For the purpose of this invention, these substances may take any fluid or solid form, for example a capsule, tablet, suppository, liquid, gel or cream. It is known that such substances can be introduced into the desired cavity by means of an applicator. Said applicator must be rigid enough to ensure it reaches the desired spot, for example it could be made of plastics or cardboard. When such an applicator is used, the substance is delivered all in one spot and the applicator is removed.

Some common drawbacks of the known applicators for intra-vaginal or intra-rectal substances administration are:
- irritation of the vaginal or rectal cavity due to the direct contact of the applicator with the vaginal or rectal cavity walls;
- the substance is applied only in a limited area of the cavity;
- when removing the applicator, part of the substance adhering to and/or following the applicator is also removed;
- the substance tends to get or flow out of the bodily cavity after application especially if the user stands in upright position.

It is an aim of the invention to retain a substance administered into the vaginal or rectal cavity of a human or animal inside said cavity for as long as possible. Also, it is another purpose of this invention to deliver said substances by means of an applicator, without irritating or minimally irritating the bodily cavity walls.

This is achieved by a system according to the present invention for delivering, distributing at several places and retaining a substance into a vaginal or an anal cavity of a human or animal, comprising:
- a web according to the invention
- an applicator comprising at least one orifice at one end for delivering said substance, wherein the end of the applicator comprising said orifice can be releasably fixed into said zone of the web by means of the two extremities of the welding regions in the proximity of the folding line of the web.

Preferably, the distance between the two extremities is 2-6 mm higher than the diameter of the applicator, for a smooth insertion and removal.

In another aspect of the invention, it is provided a web which is a morpho-anatomic flexible web intended to be introduced into a vaginal or rectal cavity of a human or animal, being provided with means of removal from said vaginal or rectal cavity,
- said web having a proximal end which corresponds to the part of the web that first contacts said cavity and a distal end which corresponds to the part of the web that last enters said cavity and
- said web comprising 2 sheets that at least partly overlap, defining an overlapping area, each of said sheets being made of the same or different non-expandable or minimally expandable atraumatic material having a thickness of less than 2mm, preferably less than 1 mm,
   wherein
- said first and second sheets are fixed to one another by means of at least 2 stopping elements, defining at least one pouch in the interior of the overlapping area for holding a substance, said pouch comprising a mouth for receiving said substance, which is continued on each side with the at least two stopping elements which extend downwards, considering the direction of insertion of the web into said cavity, forming the edges of said pouch,
   and wherein
- said web further comprises at least one blocking element for retaining an applicator in a substantially fixed position relative to the web, said blocking element having at least one blocking side which is perpendicular to the direction of insertion of the web into the vaginal or rectal cavity.

The web according to the invention is designed to be inserted into the vaginal or rectal cavity of a human or animal, by means of an applicator. Thus, a direction of insertion is defined, which is an axis crossing the web and being oriented from the end of the web that last contacts the cavity, called distal end of the web, to the end of the web that first contacts said cavity, called proximal end of the web. When looking in the direction of insertion, the top of the web or the upper part is represented by the extremity of the web that comprises its proximal point; that is the point that first contacts the cavity. At the same time, the bottom of the web or the lower part is represented by the extremity of the web that comprises its distal end; that is the point of the web that last contacts the cavity. In some embodiments, part of the web may protrude outside from the vaginal or rectal cavity. In such a situation, the bottom of the web will also comprise such part of the web. Taking into account the above definitions, then lateral sides of the web will be the left and right extremities, considering the top and bottom as defined above.

The upper edge of the web is the edge on the top of the web while the lower edge of the web is the edge situated on the bottom of the web. According to the invention, "upper", "up" or "above" will refer to a point or part situated closer to the top of the web relative to a "lower", "down" or "below" point or part, respectively.

According to the invention, "oriented upwards" means oriented towards the top of the web while "oriented downwards" means oriented towards the bottom of the web. By "moving forward" it is to be understood according the invention movement in the direction of insertion of the web into said cavity.

The web according to the invention is a "morpho-anatomic flexible web", meaning that it is made of a fabric suitable to be comfortably fitted inside the human or animal vagina or rectum, being sufficiently flexible as to follow the shape of the vagina or rectum.

The web according to the invention is made of a non-expandable or minimally expandable atraumatic material. By non-expandable or minimally expandable material is to be understood that the capacity of expansion or of dilation of the web is not like that of a traditional tampon in which the core is designed to dilate in order to be able to absorb a maximum quantity of fluid. The thickness of a sheet of the web according to the invention is of less than 2 mm, preferably less than 1 mm, even more preferably less than 0.6 mm, so it is much smaller compared to a normal tampon. Preferably, the material of the web has a basis-weight of approximately 60 g/m².

The material of the web is atraumatic, by which it is to be understood a material which may be used in contact with or to slide over or wipe a surface of a body membrane or skin without causing any injury or discomfort such as irritation, rash, pain etc., preferably a fabric with a soft carded smooth surface. The use of the atraumatic web according to the invention together with an applicator has the advantage that irritation of the vaginal or rectal cavity due to the direct contact of the applicator with the vaginal or rectal cavity walls is avoided.

Each of said first and second sheets of the web according to the invention may be made of the same or different material. When the two sheets have been fixed together, thus defining the pouch mentioned above, each sheet will have an inner side, that is the side of the sheet facing the inside of said pouch and an outer side, that is the side of the sheet facing the bodily cavity. Preferably said first and second sheets are integrally formed from the same piece of fabric and folded one over the other along a folding line.

Each of the first and second sheets is made of a material that has low absorbency, by which it is meant that it is able to absorb only very small quantities of substances as compared to a commonly known tampon used for retaining high quantities of menstrual secretions. Therefore, the web may be able to absorb and retain only very small quantities of bodily fluids. Preferably, for a web according to the invention designed to be completely housed inside the vaginal cavity of a woman, the absorption capacity is 0 - 6g, corresponding to 1 drop according to the Absorbency Rating Systems. More preferably, the web in unfolded state has a substantially rectangular shape, having a length of less than 100mm and a width of less than 60mm. For example, a preferred web according to the invention, having rectangular shape, with a length of 100mm and width of 60mm, can absorb up to 1.4 g of menstrual fluid, according to the absorbency rating system for tampons.

At the same time, when used together with a substance to be administered inside a vaginal or anal cavity, at least one of the first and second sheets of material must be permeable to said substance. This allows the substance to pass through the sheet's material and to contact the cavity in which it is introduced, producing the desired effect. The material of the web can routinely be chosen by the person skilled in the art to have a higher or lower permeability, thus allowing the desired substance to be released within a faster or slower period of time. More preferably, the material is only permeable to said substance when the substance is in molten state, which means the state of the substance subjected to the body heat, corresponding to a temperature which is higher than the temperature of said substance at the time of insertion into said cavity. Advantageously, the permeable material is hydrophilic.

At least one of the first and second sheets may be made of a material chosen from the group of a woven or non-woven textile, for example made of synthetic fibers (such as polyester, polypropylene, polyethylene, polyamide, polyacetate, polyvinyl acetate), semi-synthetic fibers (such as viscose, modal, lyocell), plant fibers (such as cotton), animal fibers (such as silk), or combinations thereof. In a preferred embodiment, the material is biodegradable, thus reducing the impact on the environment. Preferably, the material is a non-woven textile made of synthetic fibers, since such materials are atraumatic and permeable to many of the usual substances that can be found in capsules, creams, ovules, solutions etc. for inserting into the vagina or rectum and have the desired low absorbency. Also, such products may have low production costs. In a preferred embodiment, the material is also thermofusible, so that it may be welded.

In a more preferred embodiment, the atraumatic, permeable, morpho-anatomic flexible web is made of a non woven fabric with a soft carded smooth surface comprising a non-woven polyethylene/polyester bicomponent. Preferably, such material has the following properties:
- average basis weight (mass per unit area), measured with WSP 130.1 Test method, of around 59.20 g/m²,
- average tensile strength MD, representing the force per unit width which is required to rupture a sample orientated in the machine direction, measured with a Test method following WSP 110.4 using a sample width of 25.4 mm (1 inch), a clamp distance of 127 mm (5 inch) and a speed of 500 mm/min (19.7 inch/min), of around 48.86 N/inch
- average elongation at F-max MD, representing the relative increase in length at the maximum force applied on a sample orientated in the machine direction, measured with a Test method following WSP 110.4 using a sample width of 25.4 mm (1 inch), a clamp distance of 127 mm (5 inch) and a speed of 500 mm/min (19.7 inch/min), of around 38.40 %.

Preferably, the web according to the invention may be partially covered by a non-adherent material (such as gore-tex®), which prevents the web to adhere too tightly to the internal walls of the bodily cavity, thus allowing it to be removed easier and without discomfort.

The morpho-anatomic flexible web according to the invention is suitable to be inserted into the vaginal or rectal cavity of a human or animal. Thus, the person skilled in the art will understand to choose the dimensions of the web based on the dimensions of the bodily cavity where it is to be inserted.

The vagina and rectum are open cavities in the form of fibro-muscular tubes with walls that are easily distensible. The vagina is in the form of a tube having at the extremities an external opening (the vaginal opening) and an internal opening (communicating with the uterus). The rectum is in the form of a tube having at the extremities an external opening (the anus) and an internal opening (communicating with the large intestine); near the external opening it has a dilated portion, the rectal ampulla, where the web according to the invention is meant to be housed. The external openings of the vagina and rectum are substantially circular. The width (diameter) of the tubes (vagina and rectum) varies throughout their length, with the minimum width being at the external opening of the bodily cavity. For example, the human adult vagina or rectum at rest have, at the external opening, a width of about 2.5 cm.

Whatever the shape of the web when outside the bodily cavity, due to the fact that it is made of a flexible fabric, when inserted into the bodily cavity by pushing it through the substantially circular external opening, the web will collapse (deform, fold and/or twist) to pass through the external opening and then will unfold to roughly follow the shape of the cavity. Thus, when inserted into the bodily cavity (so after the web has been pushed inside the bodily cavity), the web will take a shape having a proximal end (the end of the web which is closest to the internal opening of the bodily cavity) and a distal end (the end of the web which is closest to the external opening of the bodily cavity), and will occupy a roughly tubular space. The distance from the proximal end to the distal end of the web defines the length of the web. Due to the fact that the web is made of a morpho-anatomic flexible material of a low thickness and also due to the morphology and humidity inside the bodily cavities, the web will follow the shape of the bodily cavity and will tend to maintain the shape from the moment of insertion.

Thus, the skilled person will be able to shape/choose the dimensions of the web so that when inserted into the bodily cavity, the web will preferably have a length up to the maximum length of the vagina or of the rectal ampulla. Alternatively, the web may have a length greater than the maximum length of the bodily cavity, so that part of the web protrudes outside of the bodily cavity. Preferably, the bodily cavity is a human vaginal cavity and the part of the web that protrudes outside has the dimensions suitable for use as a pantiliner. This arrangement has the effect that the web acts also as a pantiliner, absorbing the outside humidity, i.e. from urinary incontinence or perspiration. More preferably, the part of the web that protrudes outside the bodily cavity is fringed. Also preferably, the part of the web that protrudes outside may be used as means of removal for the extraction of the web from the vaginal cavity.

For example, the length of the human adult vagina at rest varies from about 5 to about 14 cm, and the length of the human adult rectal ampulla varies from about 4 to about 6 cm. Therefore, the skilled person may choose the dimensions of the web for completely inserting in the vagina of a human so that, when inserted, the web will take a shape with the length of less than 14 cm, preferably less than 12 cm, most preferably between 7 and 10 cm. For insertion in the human rectum the web will be smaller, in order to stay in the rectal ampulla, for example having a length of less than 5 cm, preferably less than 3.5 cm.

Preferably, the shape of the web when inside the bodily cavity is collapsed in regards to the shape of the same web when outside the bodily cavity, due to the fact that it is made of a flexible textile with low thickness, that is, the web has a width before insertion that is greater than the width thereof after insertion into the cavity. In addition, after insertion into the cavity, the web is maintained in place, due to its morpho-anatomic flexible material and the humidity inside the bodily cavity.

Each of the sheets of the web can have any suitable shape, preferably it may have, for example, a substantially rectangular, square, oval or circular shape.

In a preferred embodiment of a web for insertion into the human vagina, the web is formed of two equal sheets of substantially rectangular shape, with a length of less than 100 mm and a width of less than 60 mm, said dimensions being measured before insertion and when not collapsed.

The web according to the invention is provided with means of removal, for removing the web from the cavity after use. The means of removal may be, for example, at least one thread, string, strip, ribbon, wire etc., preferably a string, with a length adapted for the extraction of the web from the bodily cavity and may be made of any material which does not tear when a force necessary to extract the web from the bodily cavity is applied thereon. Preferably, rupture resistance of such means is above 50 N. A preferred means of removal is a cotton yarn having a metric yarns number Nm of 16/4.

The means of removal may be attached to the web in any suitable way known in the art that would allow a good fixation, without detaching from the web during use or removal, such as by welding, sewing, pasting, stapling, buttoning, knotting or gluing. In a preferred embodiment, the means of removal may be attached in one or more points situated on an axis of symmetry of the web substantially parallel to the direction of insertion into the vagina or rectum or forming with said direction of insertion an angle of no more than 45°.

In another example, the means of removal may be attached in one or more points of attachment that are situated on the web in a zone different than the one corresponding to the outer surface of the pouch. For example, the points of attachment are situated on a longitudinal axis that is situated such that it does not cross the pouch formed inside the web. If the means of removal were attached by welding or gluing, the welding lines would create an area that is impermeable to a substance to be delivered, such that the delivery of the substance from the pouch into the cavity would be hindered.

Preferably, said points of attachment define an area of at least 5 cm long and 6 mm wide.

In one embodiment, the means of removal are attached to the web by strangling at least one of the first and second sheets in a strangling point situated below the stopping elements relative to the proximal end of the web. In an alternative embodiment, the means of removal are attached such that the web is not creased, for example by welding the means of removal on the web. Preferably, the means of removal are attached on the outer side of one of said first and second sheet, preferably on the lower outer side of the sheet in order to facilitate an easy removal of the web. In such an embodiment, the first and second sheets will overlap on a greater surface and will be able to come into contact and adhere to each other while inside the bodily cavity, thus providing a further retention capacity of the web, as opposed to the situation when the means for removal are knotted around such a portion. In a preferred embodiment, the means of removal consist of one string, strip, wire etc. having two ends; one end being attached to the web on the outer side of said first sheet, preferably on the lower outer side thereof, and the other end being releasably attached on the outer side of said second sheet of the web. This arrangement facilitates the packaging process since there is no free end of the thread. At the same time, the thread is easy to be detached from said second sheet of the web, obtaining the free end used to remove the web from the cavity. For this and other embodiments, it would be advantageous to provide means of removal of a different color than the web, so that they would be easily distinguishable.

By stopping element it is to be understood an element that blocks a substance such as a solid, gel, cream or liquid or a bodily fluid to move forward in the direction of the flow, that is, the passage of said substance through the stopping element is obstructed. At the same time, said substance may still leave the pouch formed inside the web according to the invention, either through the web's material which is, for at least one of the two sheets, permeable to said substance, or, if desired, through an opening of said pouch.

The stopping element may be in the form of a surface or a line and may be preferably a substantially straight line or a substantially curve line. Preferably, for reasons of simplicity of the process for fabrication, the stopping elements are straight lines. Said stopping elements may comprise, for example, sewed, glued, welded lines, zones or areas or combinations thereof. A welded line or area according to the invention is obtained by welding together the first and second sheets of the web by the application of heat and pressure until they are fused together, for example by using a heat sealing machine, such as a rotary welding machine, for example a wedge welding or a hot air welding machine. According to the invention, welding is preferred, since it does not involve the use of an additional substance, such as an adhesive, that could raise compatibility and health issues when inserted into a bodily cavity. At the same time, a welded fabric will substantially retain its softness and springiness, so that the web remains atraumatic and flexible.

The stopping elements must define inside the overlapping area of the web at least one pouch, that is a container that can hold at least partly a substance for at least a limited period of time. Said pouch comprises one mouth which is opened, for receiving a substance, said mouth being continued on each side with the stopping elements which extend downwards relative to the proximal end when considering the direction of insertion of the web into a bodily cavity, that is, toward the distal end of the web, forming the edges of said pouch.

The stopping elements must be arranged such that a substance is at least partly retained into said pouch for at least a limited period of time. The intersection between the mouth and the stopping elements define the extremities of the mouth that form together an imaginary mouth line. According to the invention the extremities of the mouth are situated at the minimum distance relative to the proximal end of the web in comparison with the distance between the proximal end and any other point situated on any of the stopping elements.

The stopping elements may define a pouch that is closed or partially open.

A partially open pouch is a pouch that has at least one further opening in addition to the mouth thereof, for example in the opposite end of the stopping elements with reference to the mouth. By adjusting the dimension and position of said opening, part of the substance that is to be delivered inside the vaginal or rectal cavity flows out of the pouch through said opening, allowing a faster contact between the substance and said cavity as compared to a closed pouch web. In a preferred embodiment, said opening is configured such that the substance, at the moment of insertion of the web into a cavity, will not flow out of the pouch due to its viscosity, but when it melts as a result of the heat inside the cavity, it will easily leave the pouch through said opening and contact the cavity. In such a way, it is ensured that the substance stays in the cavity long enough to produce the desired effect, thus less substance is required for one time administration as compared to the conventional delivery of such a substance in the desired cavity without the use of a web according to the invention.

A closed pouch is a pouch that has only one opening corresponding to the mouth of the pouch. According to such an embodiment, the substance to be administered inside a vaginal or rectal cavity is held inside the pouch and can only leave the pouch through the web's material. This ensures that no substance is lost and no leakages occur when a substance is delivered inside a vaginal or rectal cavity. Also, if the pouch is designed to collect bodily fluids, then substantially no leakage of such fluids out of the pouch occurs.

In a preferred embodiment, the stopping elements form a pouch having a U-shape. In another preferred embodiment, the pouch may have a rectangular or substantially trapezoidal shape with the mouth corresponding to one of the sides of the rectangle or trapezoid respectively.

In another preferred embodiment, the edges of the pouch are formed by two stopping elements that intersect, forming a V shape which ends on one side with the mouth of the pouch. This arrangement has the advantage of being simple, cheap and versatile.

In yet another preferred embodiment, the pouch has an acute trapezoidal shape, that is, a trapezium having two substantially parallel sides of different length called bases and two lateral non-parallel sides intersecting said bases having two adjacent acute angles on its longer base. According to this embodiment, the stopping elements correspond to the lateral non-parallel sides defining the edges of the pouch, while said edges end on one side with the mouth of the pouch corresponding to the longer base of the trapezium and on the other side with an opening corresponding to the shorter base of the trapezium. Thus, a partially open pouch is formed that would still be able to hold at least partially a substance for at least a limited period of time. Preferably, when the stopping elements are not parallel to each other, they may intersect or not, and are inclined to one another at an angle of preferably between 10° and 170°, more preferably between 20° to 150°, even more preferably between 50° to 120°.

The pouch is arranged inside the web with the mouth oriented towards the proximal end of the web, that is, the 2 points of intersection between the mouth and the stopping elements are situated at the shortest distance from a point situated in the proximal end, with regards to any other points situated on the stopping elements. Advantageously, part of the web is situated below and/or laterally relative to the pouch when considering the direction of insertion of the web into a bodily cavity with the purpose of further collecting leakages from said cavity.

The web according to the invention may be inserted into the bodily cavity by using an applicator which may be a finger, or a pushing tool or an applicator for the delivery of a solid, liquid, gel or cream. The pushing tool or applicator will have a shape, and dimensions adapted to those of the bodily cavity for which it is intended and the strength necessary to be able to push the web inside the bodily cavity. The applicator may be disposable (one use-only) or reusable. The applicator may be inserted, centered or off centre, in the above mentioned pouch or in a separate, dedicated pocket.

The applicator may be made of any suitable material, such as plastic, silicon, wood, metal (such as stainless steel, titanium, or gold plated), cardboard etc. Preferably, the applicator is made of low density polyethylene PE-LD, which has low production costs, good flexibility, low weight and can be cleaned and reused.

For instance, the pushing tool may be made in the form of a tubular body. For human use, such an applicator may have a length (the height of the tube) of about 120 mm and a width (diameter of the tube) of about 11 mm.

In a preferred embodiment, the applicator is in the shape of a rod having at each of its two ends a rounded protuberance. The rounded protuberance may have for example a spherical or ovoid shape. Preferably, the rod also has a plurality of smaller protrusions situated between said ends. The rounded end of the protuberances will facilitate an easy insertion thereof into the bodily cavities. Said protuberances also have the advantage of stimulating the nerve endings in the area of the external orifices of the vaginal and rectal cavity and of the mucosa thereof, thus inducing in the area, in response, a relaxing of the muscles and a release of secretions that will facilitate the further insertion of the web. Also, preferably, the rounded protuberances at the ends of the applicator are of different diameters (dimensions), such an applicator having the advantage that it may be used for webs with different dimensions of the dedicated pocket for the applicator and/or that applicator may be inserted either with the larger or smaller end, depending on the dimensions of the bodily cavity or on the preferences of the subject. Using such an applicator is particularly advantageous for women who have vaginal atrophy, to increase the vaginal secretion during application, since such women usually have not enough vaginal secretion to facilitate the insertion of the web and the melting of an ovule or tablet. Also for the women who have vaginal atrophy, the fact that the applicator may be used in different dimensions will help reduce any difficulty and/or pain when inserting the web and applicator in the vagina.

When the applicator is used at the same time as an applicator for delivering a substance, such applicator may be any dispensing device known in the art for intra-vaginal or intra-rectal use, for example a syringe of an ordinary type, comprising a hollow cylinder and a piston capable to slide within the hollow cylinder, the end of the cylinder opposite to the piston having an orifice. The fact that the applicator is inserted in the web means that, during the insertion of the web, the applicator is covered (at least partially) by the atraumatic web according to the invention and so the applicator does not come in direct contact with the walls of the vagina or rectum; instead, the atraumatic web is placed between the dispenser and the walls of the bodily cavity. Thus, it is solved the problem of irritation of the vaginal or rectal cavity due to the direct contact of the applicator with the vaginal or rectal cavity walls.

The web according to the invention further comprises at least one blocking element for retaining an applicator in a substantially fixed position relative to the web when the applicator contacts said blocking element. Said blocking element contains at least one side, called blocking side, which is arranged substantially perpendicular to the direction of insertion of the web, so that it allows the applicator to push the web into the bodily cavity. After insertion, the applicator will be easily detachable from the blocking element and removed from said cavity by moving the applicator in the opposite direction to the one of insertion, without removing also the web.

Preferably, said blocking element may be configured also for guiding and/or housing the applicator, for example delimiting a pocket for holding the applicator.

In one embodiment, the blocking element may be represented by a folding line of the web, i.e. the line along which the material of the web is folded to result said first and second sheets of the web. When the web is inserted into the bodily cavity with the folding line at the proximal end, it is substantially perpendicular to the direction of insertion of the web, so it will retain the applicator in a fixed position relative to the web and the web will be guided inside the bodily cavity by means of the applicator. After insertion, the applicator will be easily detachable from the blocking element and removed from said cavity.

In another preferred embodiment, the blocking element is in the form of a substantially straight line, preferably a double line which corresponds to the blocking side, obtained by fixing together the first and second sheet of the web. For example, by welding the first and second sheets together, a welded line or more preferably a welded double line is obtained, representing the blocking element. More preferably, said line is continued on the sides with two other lines, substantially perpendicular on the first one or forming an angle of more than 90° with it, called guiding sides, to guide the applicator and to fixedly hold it in contact with the blocking side. Advantageously, the intersection between the mouth and the stopping elements define the extremities of the mouth that form together an imaginary mouth line. In such a preferred embodiment, the blocking side of the blocking element is situated at least 5 mm below the lowest extremity of said line, considering the direction of insertion of the web inside the bodily cavity. In such a preferred embodiment, during the insertion process, the web at the proximal end, being flexible and having no rigid support, will tend to fold over the rest of the web, in the point of contact between the applicator and the blocking element, thus covering the mouth and closing the pouch and at the same time increasing the thickness of the upper part of the web from two sheets to four sheets of material. By providing this arrangement, a better retention of the bodily fluids is achieved. At the same time, if the pouch is a closed pouch, i.e. the only opening was the mouth, after insertion into the cavity, a substance housed in it will be kept inside said pouch and will not leak outside even if the position of the human or animal will change. For example, if such a web was configured for a vaginal cavity of a woman and before insertion a substance is introduced in the web's pouch, after the insertion of the web into the vaginal cavity, the pouch will be completely closed and the substance will not leak even if the woman stands up, lays down or changes her position by 360°.

Alternatively, a pre-cut is made between the mouth and the blocking side of the blocking element, having a length corresponding to the distance from the proximal end or upper edge of the web to said blocking element. In such a situation, during the insertion process, only the part of the web situated above the applicator will fold over the rest of the web, leaving, for example, the mouth of the pouch when situated at the proximal end of the web open.

Alternatively, if it is desired that the mouth of the pouch remains open, for instance in order to more easily capture inside it the bodily secretions from the vagina, the blocking side of the blocking element may be situated laterally and at the same level with said mouth.

In another preferred embodiment, the blocking element is in the form of a pocket formed either inside the overlapping area of the web or on one of the exterior sides of the first or second sheet of the web. Such a pocket may have a substantially rectangular shape or, preferably, a substantially trapezoidal shape or any other shape suitable for a pocket. Such a shape will facilitate an easy and precise insertion of the applicator into the blocking element, said applicator remaining fixed in relation to the web during the insertion into the cavity, and will ensure that the applicator is also easily extracted after insertion, without also pulling out the web.

In yet another preferred embodiment, the blocking element has a blocking side and two guiding sides, wherein the guiding sides are not in direct contact with the blocking side, each of the upper extremities of said guiding sides being situated at a distance from the blocking side.

The web according the invention is suitable for administering a substance into a vaginal or a rectal cavity of a human or animal, said substance being substantially fluid such as a liquid, a gel, a cream or substantially solid such as a tablet, capsule or a suppository. The substance is to be administered into the cavity by means of the web's pouch such that it is placed inside the pouch before insertion of the web into said cavity. In an alternative embodiment, the substance is placed inside the pouch after insertion of the web into said cavity, for example by using a syringe containing the substance as an applicator and releasing the substance from the syringe into the pouch after insertion into the cavity. In such a situation, the blocking element and the mouth of the pouch must be both situated on the direction of insertion.

The web according to the invention may be advantageously used for the application of microbicide treatments such as gel microbicides for the treatment and prevention of HIV infection. Such microbicide gels, as for example the tenofovir gel, should be applied before and/or after the sexual intercourse. Some studies, though, have shown that the sperm renders such microbicide gels ineffective and actually increases HIV infectivity through sperm fibrilar proteins that allow the virus to agglomerate and increase its ability to attach to and infect immune cells, this effect leading to HIV infection rates being ten times higher than without the sperm and to microbicidal gels being up to twenty times less effective against the virus than when the gels are not combined with sperm. Thus, it would be highly desirable to be able to prevent this effect of the sperm rendering the microbicide gel ineffective, for example by drying out the sperm, or by using a suitable probiotic. Such probiotics are most commonly marketed in the form of tablets. The web according to the invention would be particularly advantageous for the application of said microbicide gel, since, besides the effect of retaining the gel inside the bodily cavity, it has the further advantage of inducing the sperm to dry, by wiping the sperm from the walls of the bodily cavity and fixing it on the web, and also by providing oxygen from the air outside of the cavity, thus drying out the sperm and inactivating the sperm's fibbrilar proteins thereby preventing the sperm from rendering the micobicide gel inefficient. Furthermore, the web may house a suitable probiotic as mentioned above, such as a tablet, or another active agent having the effect of inactivating the sperm's fibbrilar proteins, for example in a different pouch than the pouch containing the microbicide gel, said probiotic or active agent preventing the sperm from rendering the micobicide gel inefficient. In a particular embodiment, part of the upper edge of the web is folded over the rest of the web, thus increasing the thickness from two sheets to four sheets of material. Due to the increased thickness, when the web is inserted into the vaginal cavity, a better wiping of the sperm is achieved.

According to another embodiment, a substance according to the invention may refer to bodily fluids, either menstrual discharges or other fluids that are secreted inside the vaginal or rectal cavity and that could be collected into the web's pouch to be retained inside it until removal of the web from the cavity.

In another aspect of this invention it is provided a kit for delivering a substance into a vaginal or an rectal cavity of a human or animal, comprising:
- the web according to the invention;
- an applicator according to the invention;
- optionally, a substance for inserting into the vagina or rectum.

In another aspect of this invention are provided methods for introducing a substance into the vaginal or rectal cavity of a human or animal. A first such method for introducing a substance into the vaginal or rectal cavity of a human or animal comprises:
- inserting the substance into the pouch of a web according to the invention;
- inserting the web into the vaginal or rectal cavity of the human or animal with an applicator;
- extracting the applicator from the vagina or rectum.

Another method for introducing a fluid substance into the vaginal or rectal cavity of a human or animal comprises:
- inserting the substance into an applicator;
- inserting the applicator filled with said substance into the web according to the invention;
- inserting the web into the vaginal or rectal cavity of the human or animal with the applicator;
- releasing the substance from the applicator;
- extracting the applicator from the vaginal or rectal cavity.

In another aspect of this invention is provided a method of manufacturing the web according to the invention, comprising the steps of:
a) applying the means of removal on the material of the web, for example by welding;
b) cutting the web to the desired dimension;
c) placing two sheets of the web one above the other to form an overlapping area;
d) fixing said two sheets together with stopping elements for example by welding;
e) optionally adding at least a blocking element in the desired position.

In a method of manufacturing the web according to a preferred embodiment of the invention, said method comprises the steps of:
a) delivering a roll of material to a conveyor;
b) attaching the means of removal on the web such that areas of strong attachment alternate with areas of releasable attachment;
c) cutting the roll of material together with the means of removal at regular intervals to obtain rectangular shaped fabrics each having one area of strong attachment and one area of releasable attachment of step b;
d) folding each rectangular fabric of the preceding step by lifting it along a folding line that represents its middle width, thus obtaining two overlapping sheets of the web;
e) welding together said two sheets of the web along desired lines or areas defining a closed pouch and next to it a blocking element.

Optionally, a further step may be added before step d) to partially cut the roll of material along the line that represents the middle width of said rectangular fabric. This has the advantage that will provide a clear indication of where the applicator should to be inserted. Furthermore, it would be preferable that the means of removal are attached over the area of the web where said cut is performed; this has the advantages that, on the one hand, the attachments (for example by welding) of the removal means will be made in a different area of the web than the pouch, thereby not affecting in any way the proprieties of the web material in the pouch area, and, on the other hand, it has the advantage that, in order to insert the applicator, the removal means must be released from the releasable attachment, thus preventing the user from inserting the web without first releasing the removal means.

Also, another purpose of the invention is to deliver and distribute a substance to be administered to a human or animal in more than one spot in the vaginal cavity or rectum of humans or animals.

This is achieved by the web according to the invention, which is a morpho-anatomic flexible web intended to be introduced into a vaginal or rectal cavity of a human or animal, being provided with means of removal from said vaginal or rectal cavity, wherein
- a first portion of the web is folded over a remaining second portion of the web along a folding line,
- the first and second portions of the web are maintained in a folded position by a plurality of welding regions, thereby :
   i) defining a retaining pouch having an opening in the region opposite to the folding line,
   ii) also defining together with the folding line a zone with two further openings at the extremities of the folding line,
- the extremities of the welding regions in the proximity of the folding line are disposed such that the pouch and the zone can receive an applicator for the delivery of a substance in said zone, wherein the extremities of the welding regions in the proximity of the folding line are disposed such that at least a fraction of the liquid, gel or cream delivered into said zone by the applicator 30 is guided through said two further openings.

The two extremities of the welding regions in the proximity of the folding line are disposed to allow the insertion of the applicator and are situated at a distance between each other to substantially match the diameter of said applicator. For a smooth insertion and removal, the distance between the two extremities is 2-6 mm bigger than the diameter of the applicator.

The syringe can be introduced into the web via its end comprising the orifice which allows the delivery of the substance, being releasably fixed into the zone in the proximity of the folding line by means of the two extremities of the welding region. When introduced into vaginal or anal cavity, said orifice is covered by the web, such that in case the fluid substance is delivered under a relatively high pressure, the flush of substance will be guided laterally by the web's shape, thus avoiding the penetration of the substance in undesired places, for example into the uterus. Because of the web's fluid retaining capability and its anatomical flexible shape, the fluid substance may be delivered also when the person is in a standing position, as, after delivery and distribution, the fluid substance will not immediately leak out of the bodily cavity.

The invention will be better understood from the detailed description given below and by reference to the drawings, in which:
Fig. 1 represents a front view of different embodiments of the web according to the invention having a closed pouch;
Fig. 2 represents a front view of different embodiments of the web according to the invention having a partially open pouch;
Fig. 3 represents a front view of an embodiment of the web according to the invention having two pouches;
Fig. 4 represents a front view of an applicator according to some embodiments of the invention;
Fig. 5 represents a sectional view of different embodiments of a web according to the invention together with an applicator;
Fig. 6 represents sectional views of one of the embodiments according to the invention, with the web taking different shapes before and after insertion in a vaginal cavity
Fig. 7 represents a sectional view of a rectal cavity with a web according to one of the embodiments of the invention;
Fig. 8 represents a front view of another embodiment of the web according to the invention having guiding sides for the applicator and removal means;
Fig. 9 represents a front view of another embodiment of the web according to the invention having guiding sides for the applicator and removal means, together with an applicator;
Fig. 10 represents a front view of another embodiment of the web according to the invention, having guiding sides for the applicator and removal means applied on the web;
Fig.11-13 represent the fluid substance flow directions when using the web according to another embodiment of the invention.

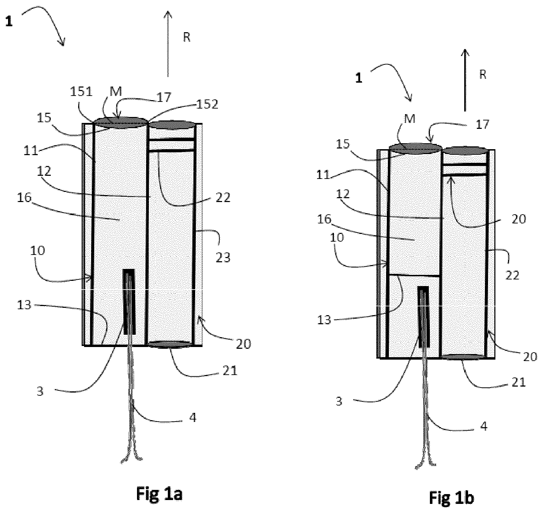

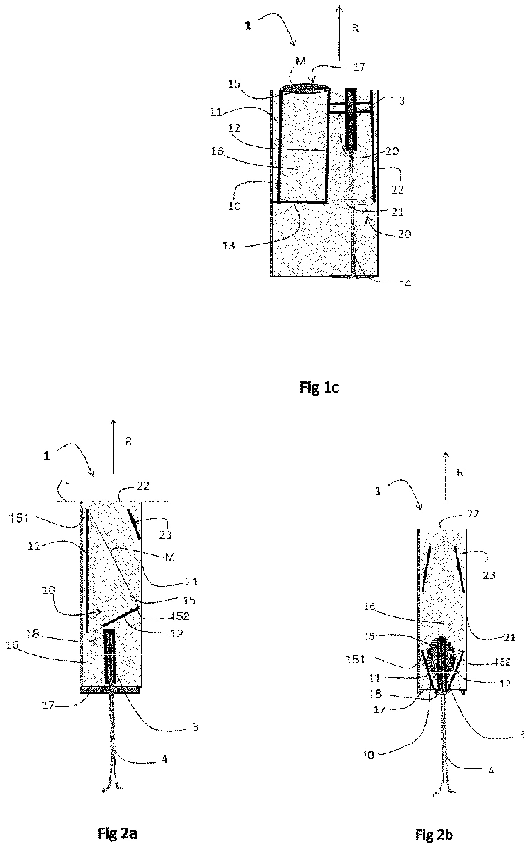

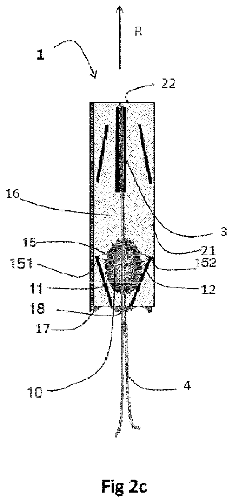

Fig 1 and 2 depict several embodiments of a web 1 according to the invention to be introduced into the vaginal or rectal cavity of a human or animal, comprising a first sheet 16 and second sheet 17, each having a substantially rectangular shape. In a preferred embodiment, the web 1 has a length of less than 100 mm, preferably less than 100 mm, and a width of less than 60 mm, the first and second sheets 16,17 being substantially equal and fully overlapped, the proximal end 5 of the web 1 being situated on a first short side of the rectangle and the distal end 6 of the web being situated on the second short side of the rectangle.

In the overlapping area of the web 1 which is created by overlapping the first and second sheets 16, 17, a pouch 10 is defined, having a mouth 15 which is open, to allow a substance to be introduced into said pouch, said mouth 15 being oriented upwards, considering the direction of insertion R of the web into said cavity. In a preferred embodiment according to fig. 1, said mouth 15 is situated at the upper edge of the web, such that it remains in contact with the exterior and can therefore receive into the pouch 10 bodily fluids from said cavity.

In an alternative embodiment according to fig. 2, the mouth 15 of the pouch 10 is housed inside the overlapping area of the web 1 and the upper edge of the web 1 is closed. For example, the first and second sheets 16,17 of atraumatic material are integrally formed and folded one over the other upon a folding line, which defines the upper edge of the web 1.

The mouth 15 is continued on each side with a stopping element 11,12 which extend downwards, considering the direction of insertion R of the web 1 into said cavity. In a preferred embodiment (fig 1a, 1b, 1c) such stopping elements 11, 12 are parallel to each other and are connected by means of a third stopping element 13, such that the pouch 10 is completely closed, meaning that the only opening of the pouch 10 is the mouth 15. The pouch 10 can have the same length as the web (fig 1a) or lower (fig 1b, 1c).

According to an alternative embodiment (fig 2a, 2b, 2c) the pouch 10 is partially open, this meaning that at least a further opening 18 is provided in said pouch 10. The stopping elements 11,12 keep together the first and second sheets 16,17 of the web 1, defining the edges of the pouch 10.

The intersection between the mouth 15 and the stopping elements 11, 12 define the extremities of the mouth 151 and 152 respectively, that form together an imaginary mouth line M.

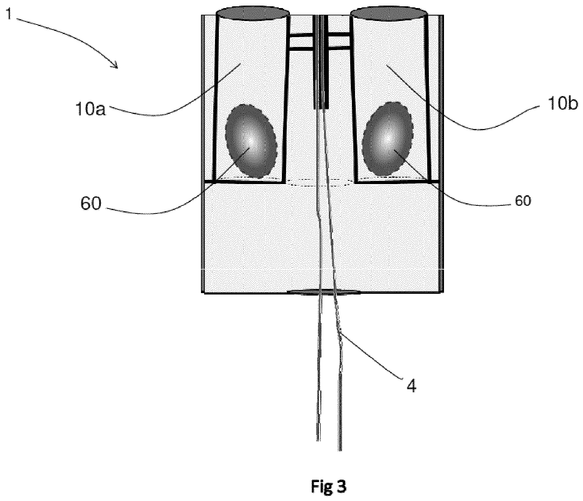

In yet another preferred embodiment (fig 3), the web 1 comprises two pouches 10a and 10b, for holding advantageously two different substances.

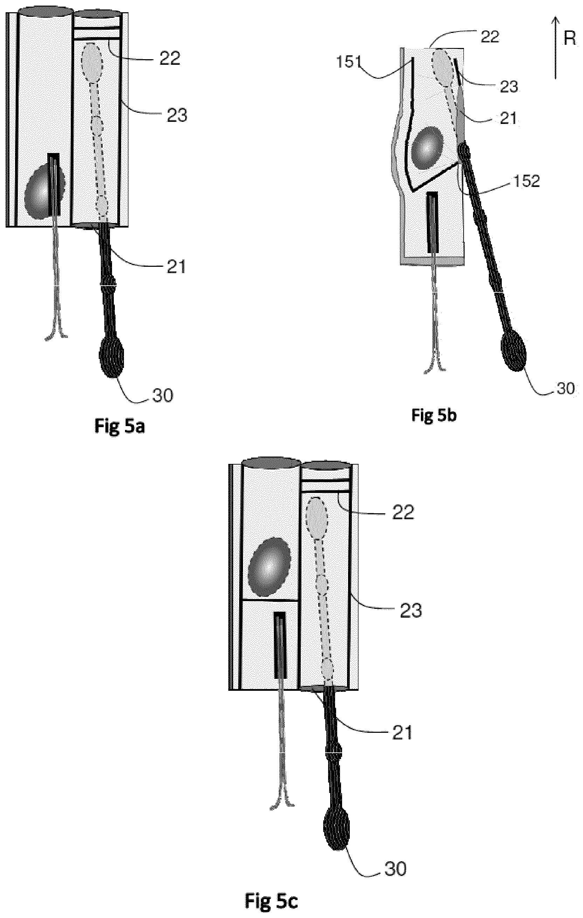

As can be seen from fig. 1, 2 and 5, the web 1 according to the invention further comprises a blocking element 20 for retaining an applicator 30 (fig 5) in a substantially fixed position relative to the web 1. The blocking element 20 has at least one side 22 which is perpendicular to the direction R of insertion of the web 1 into said cavity.

According to a preferred embodiment, said blocking element 20 is situated laterally from the pouch 10 (fig. 1, 5a, 5c). Preferably, the side 22 is in the form of one straight line that fixes the first and second sheets 16,17 together, for example by welding, more preferably in the form of two such straight lines, one on top of the other, considering the direction of insertion R, to improve the strength of the blocking element 20. More preferably, the side 22 of the blocking element 20 is situated at least 5 mm below the lowest extremity 151, 152 of the mouth line M, considering the direction of insertion R. In an alternative embodiment, a pre-cut is made between the mouth 15 and the side 22 of the blocking element 20, having a length corresponding to the distance from the upper edge of the web 1 to said side 22.

In yet a more preferred embodiment, the blocking element 20 has sides 23 for guiding and housing an applicator 30 (fig 5a, 5c). Preferably, the blocking element 20 and the pouch 10 have one edge in common which serves both as a stopping element 12 of the pouch 10 and at least partly as a side 23 of the blocking element 20 for guiding and housing an applicator 30 (fig 1, 5a, 5c).

Alternatively, as can be seen from fig 2 and fig 5b, the first and second sheets 16,17 of the web 1 are integrally formed and folded one over the other upon a folding line L, which defines the upper edge of the web 1 and which preferably corresponds to the side 22 of the blocking element 20. Further sides 23 may be provided on the web 1 to guide the applicator 30 towards the side 22.

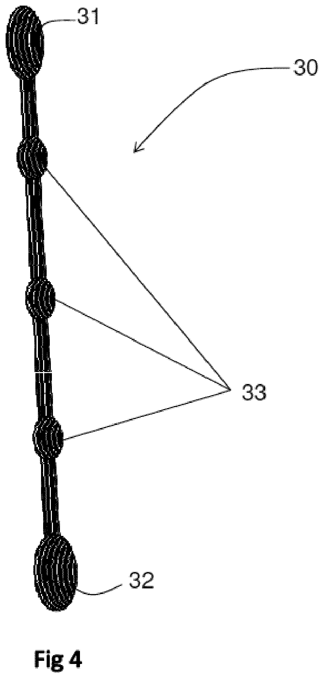

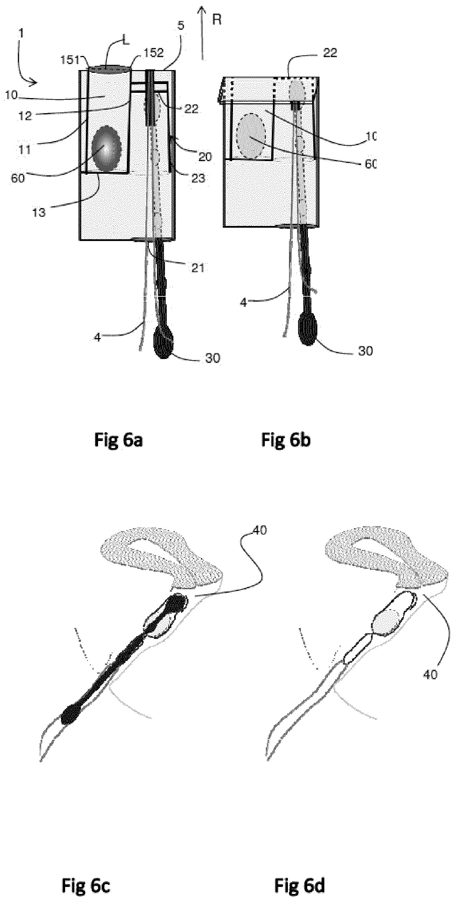

An applicator 30 according to a preferred embodiment is depicted in fig. 4 and has the shape of a rod, having two ends 31, 32 with rounded protuberance, such as spherical or ovoid protuberances. Said ends 31, 32 may have the same or different shape and dimension. More preferably, there are provided on said rod a plurality of protrusions 33, advantageously smaller than said protuberances at the ends 31, 32. Fig 5 and 6 represent different ways of using a system according to the invention comprising a web 1 and an applicator 30. When an applicator 30 is inserted into the blocking element 20 via an opening 21, it advances, preferably guided by the guiding elements 23, until the applicator 30 contacts the side 22 of the blocking element 20. The applicator 30 is blocked by the side 22 from moving forward, considering the direction of insertion R, such that said applicator is retained in a substantially fixed position relative to the web 1.

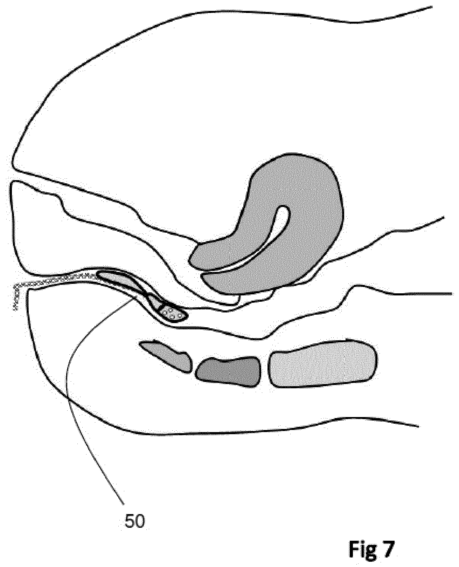

When the applicator contacts the side 22 of the blocking element, the system is ready to be inserted into the desired cavity, for example the vaginal cavity 40 (fig 6c, 6d) or rectal cavity 50 (fig 7).

According to a preferred embodiment as can be seen in fig. 6, a system is provided comprising a web 1 according to the invention and an applicator 30. The web 1 comprises a pouch 10 with a mouth 15 situated on the upper edge of the web 1 such that it has direct contact with the exterior of the web 1 and a blocking element 20 arranged laterally and in contact with one edge 12 of said pouch, with the side 22 situated below the mouth line L, preferably more than 5mm below said mouth line L. A substance 60 is introduced into the pouch 10 of the web 1 before insertion of the web 1 into the desired cavity. The applicator 30 is introduced through the opening 21 and advances, guided by the guiding sides 23, toward the side 22 of the blocking element 20. When the applicator 30 contacts the side 22 of the blocking element 20, it cannot move forward, and remains substantially fixed relative to the web 1. When the system is to be inserted into the cavity, for example the vaginal cavity 40 (fig 6c), the web 1 first contacts the cavity 40 at the proximal end 5, corresponding in this case to the part of the upper edge of the web 1 that is above the side 22 of the blocking element 20. Due to the flexibility of the web 1 at the proximal end 5, part of the web 1 comprising its upper edge will fold over the rest of the web 1, at a site corresponding to the side 22 of the blocking element 20, thus covering also the mouth 15 of the pouch 10 (fig 6b). According to this embodiment, the pouch 15 becomes completely closed when it enters the vaginal cavity 40 and remains completely closed inside said cavity also after removal of the applicator 30 (fig 6c, 6d). In such a situation, the substance 60 will not leak even if the user, for example a woman, stands up, lays down or changes her position by 360°. In such a situation, the substance 60 will be released through the at least one of the first and second sheets 16, 17 forming the walls of the pouch 10, thus reaching the desired administration spots.

The web 1 is further provided with removal means 4 (fig 1, 2, 3, 5 and 6) which are attached to the web 1 in a conventional manner, preferably on an outer side of the web 1, more preferably attached such that the web is not creased, for example by welding the removal means 4 on the web 1 over a welding area 3. The removal means 4 may be attached in any part of the web, for example in the lower outer side of the web 1 (see fig. 1a, 1b, 2a, 2b) or in the upper outer side of the web (fig. 1c). At the same time, the removal means may be attached in an area corresponding to the pouch 10 (fig 1a, 2b) or alternatively in an area which does not overlap with the pouch 10, either below said pouch (fig 1b, 2a), above said pouch (fig. 2c) or laterally at the same level with said pouch 10 (fig 1c). Alternatively, the web 1 comprises two pouches 10a and 10b and the removal means 4 are situated between the pouches 9 (fig 3).

The pouch 10 can have the same length as the web (fig 1a) or lower (fig 1b, 1c).

By means of a system according to the invention, comprising a web 1 and an applicator 30, either a substantially fluid or a substantially solid substance 60 is delivered into the vagina 40 or rectal ampulla 50 by placing said substance 60 inside the pouch 10 of the web 1 according to the invention. Preferably, a substantially solid substance 60 such as a capsule or a suppository will be placed inside said pouch 10 before the insertion of the web 1 into the bodily cavity 40, 50; a substantially fluid substance such as a liquid, gel or cream may be placed inside the pouch 10 either before the insertion of the web 1, and then the web 1 together with the substance 60 may be inserted into the bodily cavity 40, 50 by means of the applicator 30, or during/after the insertion of the web 1, by using a applicator 30 such as a syringe (not shown) as described above. For example, a plastic syringe filled with the substance 60 can be used as the applicator 30, by inserting the syringe in the blocking element 20. According to this embodiment, the blocking element 20 has the side 22 substantially above the mouth 15 of the pouch 10, considering the direction of insertion R, such that when the applicator 30 is inside said cavity, by releasing the substance 60 from said applicator 30, said substance will flow and will be collected inside the pouch 10 via the mouth 15.

According to a preferred embodiment, the width of the mouth 15 of the pouch 10 is 2-6 mm higher than the diameter of the applicator 30. Preferably, the mouth 15 of the pouch 10 is placed below and sufficiently close to the blocking element 20 (for instance less than 3 cm below, preferably less than 2 cm), so that when introduced into vaginal or rectal cavity, the fluid substance 60 being delivered under a relatively high pressure, the flush of substance will be guided laterally by the side 22 and preferably by the sides 23 of the blocking element 20, thus avoiding the penetration in undesired places, for example into the uterus. The applicator 30 will then be retracted from the web 1, and the remaining substance 60 will be then housed into said pouch 10, which will allow for its release into the bodily cavity 40, 50 and will slow its flowing out of the bodily cavity. Because of the web's fluid retaining capability and its anatomical flexible shape, the fluid substance 60 may be delivered also when the person is in a standing position, as after delivery and distribution, the fluid substance will not immediately leak out of the bodily cavity 40, 50.

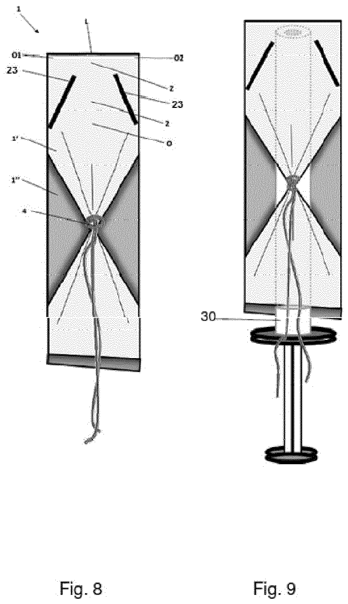

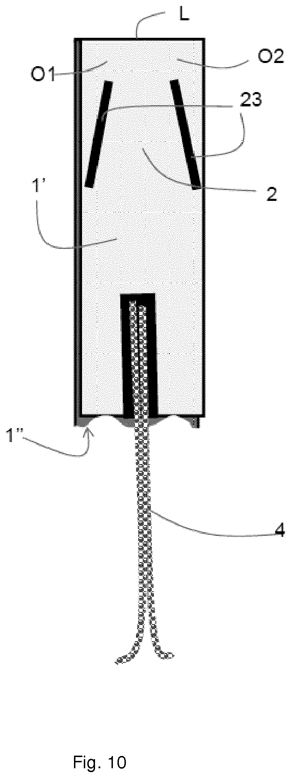

According to another embodiment of fig 8-13, the morpho-anatomic web 1 has preferably a substantially rectangular shape, being folded along one folding lines L, at one end. Said web 1 is maintained in folded position by a plurality of welding regions 23 which correspond to the guiding sides of the blocking element 20, while the folding line L corresponds to the blocking side 22 of the blocking element 20. The welding regions may be welding lines, welding points or welding ellipses.

A first portion 1' of the web 1 is folded over a remaining second portion 1" of the web 1 along a folding line L, the first 1' and second 1" portions of the web 1 are maintained in a folded position by a plurality of welding regions 23, thereby defining a retaining pouch 2 having an opening O in the region opposite to the folding line L, and also defining together with the folding line L a zone Z with two further openings O1, O2 at the extremities of the folding line L, the extremities of the welding regions 23 in the proximity of the folding line L are disposed such that the pouch 2 and zone Z can receive a applicator 30 or applicator 30 (fig 9) for the delivery of a substance which may be any of a liquid, gel or cream, into the zone Z. The web 1 is also provided with means of removal 4. The extremities of the welding regions 23 in the proximity of the folding line L are disposed such that at least a fraction of the liquid, gel or cream delivered into the zone Z by the applicator 30 is guided through the two further openings O1, O2.

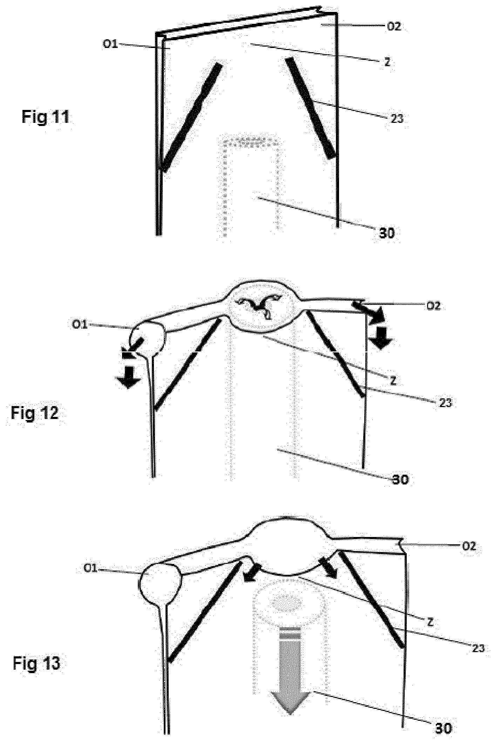

This arrangement allows the delivery of the desired substance in more than one spot inside the vaginal or anal cavity. As can be seen from figure 12, when released from the dispensing device, the substance to be delivered will be guided through the openings O1 and O2 and also through the pouch 2, once the applicator 30 is removed. Because of the pressure, the two regions of the web are pressed together, keeping the substance inside the pouch 2 and limiting the leakage. Moreover, the web's material may become impregnated with said substance allowing the delivery of the substance through the web 1, when in contact with the cavity walls. Preferably, the welding zones 23 are symmetrically placed in respect to the longitudinal axis of the first portion 1' of the web 1.

The web 1 further comprises means of removal 4 which may consist of one string attached to the second portion 1" of the web 1, such that the attachment region of the string 4 is situated in the central zone or near either of the end regions of the second portion 1", the width of the second portion 1" in the attachment region is significantly smaller than the width of the second portion 1" in either end region. According to a preferred embodiment, as can be seen from figures 8-10, the welding lines 23 are inclined. Preferably, the first portion 1' of the web 1 overlaps the second portion 1" of the web 1 over a length of at least 4.5 cm, preferably between 6-10 cm. These dimensions ensure a very good retention of the substance inside the pouch 2 and also an effective area of the web is impregnated with the fluid substance that is further delivered to an effective area of the bodily cavity walls. It is also preferred that for each welding line 23, the welding line extremity that is closer to the folding line L is at a distance of 8-12 mm from the folding line L, preferably at a distance of 10 mm. These dimensions ensure an adequate flow of the fluid substance through the openings O1 and O2.

Preferably, the web 1 is folded along a folding line L situated such that the two resulting web portions 1', 1" are substantially equal. When the two portions 1' and 1" are substantially equal, the distribution of the fluid substance to the bodily cavity walls is more effective due to the increased area of the web 1.

In another preferred embodiment, at least one notch (not shown), preferably two notches are provided, said notches being situated in the central zone of the folding line L and having a length of preferably 1-5 mm. The notches allow a limited quantity of the fluid substance to exit the zone Z in a direction perpendicular to the folding line L.

Figures 12 and 13 show the flow directions of the fluid substance delivered by the applicator 30, when using the web 1 according to figures 8-10. The fluid substance under pressure will be guided towards the web, thus avoiding the fluid substance to directly contact or enter the uterus.

The applicator 30 movement and fluid substance flow directions when using the web 1 according to an embodiment of the invention is shown in figures 11-13. The applicator 30 is pushed into the pouch 2 towards the folding line L and towards the zone Z (fig 11). The applicator 30 reaches the zone Z and delivers the fluid substance under pressure. Due to the pressure and to the welding lines emplacement, part of the fluid substance is guided through the openings O1 and O2, in the directions indicated by the arrows (fig 12). After delivery of all the quantity of fluid substance the applicator 30 is retracted. The amount of fluid substance that was not guided through the openings O1 and O2 remains in the pouch 2 and in time will impregnate the whole area of the web 1 and from there it will be transferred to the bodily cavity walls.

As can be seen from figures 11-13, upon receiving the fluid substance, the web 1 changes its shape, from a substantial flat one to a bloated shape, having a T shape section. Said bloated shape is advantageous in the sense of increasing the contact area between the web 1 and the bodily cavity walls. More precisely, the web in bloated state will mold on the bodily cavity walls.

The permeability of the web according to the invention to different substances was tested. The web 1 according to the invention was made from a nonwoven fabric with a soft carded smooth surface, comprising polyethylene and polyester. Tests were performed in order to assess the permeability of this material to substances in both solid and liquid form, using dipropylphthalate as solid substance and dicyclohexylphthalate as liquid substance. The rate of dissolving of these substances in colza oil was calculated when the substances were placed directly in the colza oil and compared to the rates of dissolving when the substances were placed first in the web according to the invention and then the web with the substance was put in colza oil. Also, a blank (web without substance) was tested to verify that the web 1 itself did not contain any of the substances. The recovery rates of the two substances in the colza oil after 2h, 4h, and 8h are depicted in the following table, showing that the material is highly permeable to these substances, (for the solid substance the dissolving rate was even greater when the substance is placed in the web than if it is put directly in the oil) and therefore it can be used for the delivery of substances placed inside a pouch made of such a material to the outside of such a pouch, for example into a bodily cavity.

| | Content of dipropylphthalate recalculated (µg/mL) | Content of dipropylphthalate theoretical (µg/mL) | Recovery rate (%) |
|---|---|---|---|
| RN16-23531 sample after 2h | 21.5 | 18.09 | 118.8 |
| RN16-23531 sample after 4h | 20.0 | | 110.5 |
| RN16-23531 sample after 8h | 21.0 | | 115.9 |

| | Content of dicyclohexylphthalate recalculated (µg/mL) | Content of dicyclohexylphthalate theoretical (µg/mL) | Recovery rate (%) |
|---|---|---|---|
| RN16-23531 sample after 2h | 16.7 | 17.23 | 92.0 |
| RN16-23531 sample after 4h | 16.3 | | 89.9 |
| RN16-23531 sample after 8h | 15.1 | | 83.7 |

The present disclosure is further described by way of the following numbered clauses:
1. Morpho-anatomic flexible web (1) intended to be introduced into a vaginal or rectal cavity (40,50) of a human or animal, being provided with means of removal (4) from said vaginal or rectal cavity (40,50),
   - said web (1) having a proximal end which corresponds to the part of the web (1) that first contacts said cavity (40,50) and a distal end which corresponds to the part of the web (1) that last enters said cavity (40,50) and
   - said web (1) comprising two sheets (16,17) that at least partly overlap, defining an overlapping area, each of said sheets (16,17) being made of the same or different non-expandable or minimally expandable atraumatic material having a thickness of less than 2mm, preferably less than 1mm,
      wherein
   - said first and second sheets (16,17) are fixed to one another by means of at least two stopping elements (11,12,13), defining at least one pouch (10) in the interior of the overlapping area for holding a substance (60), said pouch (10) comprising a mouth (15) for receiving said substance (60), which is continued on each side with one of the at least two stopping elements (11,12) which extend downwards, considering the direction of insertion of the web (1) into said cavity (40,50), forming the edges of said pouch (10),
      and wherein
   - said web (1) further comprises at least one blocking element (20) for retaining an applicator (30) in a substantially fixed position relative to the web (1), said blocking element (20) having at least one blocking side (22) which is perpendicular to the direction of insertion of the web (1) into the vaginal or rectal cavity (40,50)
2. The web (1) of clause 1 wherein the atraumatic material is a woven or non-woven textile comprising synthetic fibers, semi-synthetic fibers, plant fibers, animal fibers or combinations thereof.
3. The web (1) of clause 2 wherein the atraumatic material is hydrophilic.
4. The web (1) according to clause 2 or 3 wherein the atraumatic material comprises polyester, polypropylene, polyethylene, polyamide, polylacetate, polyvinyl acetate, viscose, modal, lyocell, cotton, silk, or combinations thereof.
5. The web (1) according to any preceding clause wherein the atraumatic material is a nonwoven fabric with a soft carded smooth surface, comprising polyethylene and polyester.
6. The web (1) according to any preceding clause wherein the atraumatic material has a basis-weight of approximately 60 g/m².
7. The web (1) according to any preceding clause wherein the atraumatic material, having dimensions suitable to be completely housed inside the vaginal cavity (40) of a woman, has an absorption and/or retention capacity of less than 6g.
8. The web (1) according to clause 7 having in unfolded state a substantially rectangular shape, with a length of less than 100mm and a width of less than 60mm.
9. The web (1) according to any previous clause wherein the atraumatic material has an absorption capacity of less than 0.3g/cm2, preferably less than 0.21g/cm2.
10. The web (1) according to any preceding clause wherein the first and second sheets (16,17) are made of the same atraumatic material.
11. The web (1) according to any preceding clause wherein the first and second sheets (16,17) of atraumatic material are integrally formed and folded one over the other upon a folding line (L).
12. The web (1) according to any preceding clause having dimensions which are designed in such a way as to allow the web (1) to be completely housed inside the vaginal or rectal cavity (40,50) of said human or animal.
13. The web (1) of clauses 1-11 having dimensions which are designed in such a way that, after insertion, part of the web (1) protrudes outside of the bodily cavity.
14. The web (1) according to clause 13 wherein the part of the web (1) that protrudes outside has the dimensions suitable for use as a pantiliner.
15. The web (1) according to any of preceding clauses having a substantially rectangular, square, oval or circular shape.
16. The web (1) according to any preceding clause having a rectangular shape with a length of less than 100mm and a width of less than 60mm.
17. The web (1) according to any preceding clause wherein the means of removal (4) is in the form of a string.
18. The web (1) according to any preceding clause wherein the means of removal (4) are attached to the web (1) on the outer side of the first sheet (16) of the web (1), preferably on the lower outer side of said sheet (16).
19. The web (1) of any preceding clause, wherein the means of removal (4) are attached such that the web (1) is not creased.
20. The web (1) of any clause 17 to 19 wherein the means of removal (4) are attached by welding, sewing, pasting, stapling, buttoning or gluing.
21. The web (1) of any of clauses 17-20 wherein the means of removal (4) are also releasably attached on the outer side of the second sheet (17) of the web (1).
22. The web (1) of any of clauses 17-21 wherein the means of removal (4) are attached in one or a plurality of points situated outside the area of said pouch (10).
23. The web (1) according to clause 17 wherein the means of removal (4) are attached to the web (1) by strangling at least one of the first and second sheets (16,17) in a strangling point situated below the pouch (10) relative to the direction of insertion.
24. The web (1) according to any of the preceding clauses wherein each of the stopping elements (11,12,13) is in the form of a substantially straight or substantially curved line.
25. The web (1) according to any preceding clause wherein the pouch (10) is closed.
26. The web (1) according to clause 25 wherein the stopping elements (11,12,13) form a pouch (10) having a U-shape, a rectangular or trapezoidal shape with the mouth (15) corresponding to one of the sides of the rectangle or trapezoid respectively.
27. The web (1) according to clause 25 comprising two stopping elements (11,12) that form a pouch (10) having a "V" shape.
28. The web (1) according to clause 27 wherein the stopping elements (11,12) form an angle of between 10° and 170°, preferably 20° to 150°, even more preferably 50° to 120°.
29. The web (1) according to any of clauses 1 to 24 wherein the pouch (10) is a partially open pouch.
30. The web (1) according to clause 29 wherein a further opening (18) of the pouch (10) is situated opposite to the mouth (15) of the pouch (10) relative to the stopping elements (11,12,13) or on a lateral side of the pouch (10).
31. The web (1) according to any preceding clause comprising two pouches (10a, 10b).
32. The web (1) according to any preceding clause wherein the blocking side (22) of the blocking element (20) is represented by the folding line (L) of the web (1).
33. The web (1) according to any preceding clause wherein the at least one blocking element (20) is in the form of a line or a double line situated one on top of the other, for example a welded line or a welded double line, obtained by fixing together said first and second sheets (16,17).
34. The web (1) according to any of clauses 1-33 wherein the at least one blocking element (20) contains guiding sides (23) for guiding and/or housing an applicator (30).
35. The web (1) according to clause 34 wherein the at least one blocking element (20) has the shape of a pocket to receive said applicator (30).
36. The web (1) according to any preceding clause wherein the blocking side (22) of the blocking element (20) is situated at least 5mm below the lowest extremity (151,152) of the mouth (15) relative to the direction of insertion of the web (1) inside the cavity (40,50), more preferably 1cm below said extremity (151,152).
37. The web (1) according to any preceding clause comprising an opening (21) for inserting an applicator (30) into the blocking element (20) arranged such that an axis connecting any point of said opening (21) with any point of the blocking side (22) of the blocking element (20) will not intersect the mouth (15) of the pouch, in particular the mouth line (M).
38. A system comprising a web (1) according to any of clauses 1-37 and an applicator (30).
39. The system according to clause 38, wherein the applicator (30) is in the shape of a rod.
40. The system according to clause 39, wherein the applicator (30) has a body and two ends (31,32), each of the two ends having a rounded protuberance, such as spherical or ovoid.
41. The system according to clause 40, wherein the two rounded protuberances of the ends (31, 32) of the applicator (30) have different dimensions.
42. The system according to clause 40, wherein the applicator (30) further has on the rod a plurality of protrusions (33) smaller than said protuberances at the ends (31,32).
43. The system according to clause 38, wherein the applicator (30) is a dispensing device, such as a syringe.
44. Method for administering a substance (60) into the vaginal or rectal cavity (40,50) of a human or animal comprising:
   - introducing the substance (60) into the pouch (10) of the web (1) according to clauses 1-37;
   - inserting said web (1) into the vaginal or rectal cavity (40,50) of the human or animal with an applicator (30);
   - extracting the applicator (30) from the vaginal or rectal cavity (40,50), without removing also the web (1).
45. Method for administering a fluid substance (60) into the vaginal or rectal cavity (40,50) of a human or animal, comprising:
   - introducing a fluid substance (60) into an applicator (30);
   - inserting the applicator (30) filled with said substance (60) into the web (1) according to any of clauses 1-37;
   - inserting the web (1) into the vaginal or rectal cavity (40,50) of the human or animal with the applicator (30);
   - releasing the substance (60) from the applicator (30);
   - extracting the applicator (30) from the vaginal or rectal cavity, without removing also the web (1).
46. Flexible web (1) for distributing at several places and for retaining a liquid, a gel or a cream inside the vaginal or anal cavity of a human or animal, said web (1) being provided with means of removal (4) from said vaginal or anal cavity, wherein
   - a first portion (1') of the web (1) is folded over a remaining second portion (1") of the web (1) along a folding line (L),
   - the first (1') and second (1") portions of the web (1) are maintained in a folded position by a plurality of welding regions (23), thereby :
      i) defining a retaining pouch (2) having an opening (O) in the region opposite to the folding line (L),
      ii) also defining together with the folding line (L) a zone (Z) with two further openings (O1, O2) at the extremities of the folding line (L),
   - the extremities of the welding regions (23) in the proximity of the folding line (L) are disposed such that the pouch (2) and zone (Z) can receive a dispensing device (D) for the delivery of said liquid, gel or cream into the zone (Z),
   characterized in that
   the extremities of the welding regions (23) in the proximity of the folding line (L) are disposed such that at least a fraction of the liquid, gel or cream delivered into the zone (Z) by the dispensing device (D) is guided through the two further openings (O1, O2).
47. Flexible web (1) according to clause 46, wherein the welding zones (23) are symmetrically placed in respect to the longitudinal axis of the first portion (1') of the web (1).
48. Flexible web (1) according to clause 47, wherein the welding zones (23) are welding lines.
49. Flexible web (1) according to clause 46, wherein the welding zones (23) are welding points or welding ellipses disposed in two groups, each group comprising an equal number of collinear points or ellipses.
50. Flexible web (1) according to clause 48, wherein the flexible web (1) is provided with two welding lines (3).
51. Flexible web (1) according to clause 50, wherein the welding lines (3) are parallel to each other.
52. Flexible web (1) according to clause 50, wherein the welding lines (3) are inclined.
53. Flexible web (1) according to clause 49, wherein the two groups of welding points or welding ellipses (3) are disposed along two parallel lines.
54. Flexible web (1) according to clause 49, wherein the two groups of welding points or welding ellipses (3) are disposed along two inclined lines.
55. Flexible web (1) according to clause 54, wherein the pouch (2) and/or zone (Z) is/are initially in a bloated state.
56. Flexible web (1) according to clauses 46-55, wherein a third portion (1"') of the web (1) is folded over the second portion (1") of the web (1) along a second folding line (L') opposite to the first folding line (L), such that the first portion (1') and the third portion (1"') are identical in terms of area and means for maintaining the respective portions in folded position.
57. Flexible web (1) according to clauses 46-56 wherein the means of removal consist of one string (4) attached to the second portion (1") of the web (1), such that:
   i) the attachment region of the string (4) is situated in the central zone or near either of the end regions of the second portion (1")
   ii) the width of the second portion (1") in the attachment region is significantly smaller than the width of the second portion (1") in either end region.
58. Flexible web (1) according to clause 52, wherein the first portion (1') of the web (1) overlaps the second portion (1") of the web (1) over a length of at least 4.5 cm, preferably between 6-10 cm.
59. Flexible web (1) according to clause 58, wherein for each welding line (23), the welding line extremity that is closer to the folding line (L) is at a distance of 8-12 mm from the folding line (L), preferably at a distance of 10 mm.
60. Flexible web (1) according to clauses 58-59, wherein the distance between the welding line extremities that are the closest to the longitudinal line (L) is between 0.5-15 mm greater than the diameter of the dispensing device (D), preferably between 1-3 mm.
61. Flexible web (1) according to clauses 58-60 wherein at least one notch, preferably two notches (N) are provided, said notches (N) being situated in the central zone of the folding line (L), and having a length of 1-5 mm.
62. Flexible web (1) according to clauses 58-61 wherein the means of removal consist of one string (4) attached to the first portion (1') of the web (1), such that:
   - the attachment region of the string (4) is situated in the central zone or near either of the end regions of the second portion (1")
   - the width of the second portion (1") in the attachment region is significantly smaller than the width of the second portion (1") in either end region.
63. Flexible web (1) according to clauses 58-62, wherein the means of removal consist of one string (4) attached to the first portion (1') of the web (1), such that:
   - the distance between the attachment point of the string (4) and the folding line (L) is at least 6 cm.
   - the width of the first portion (1') in the attachment region is significantly smaller than the width of the first portion (1') in the end region.
64. Flexible web (1) according to clauses 58-61 wherein the means of removal consist of two strings (4) attached to the first portion (1') and respectively to the second portion (1") of the web (1), such that:
   - the attachment regions of the strings (4) are situated in the central zone or near either of the end regions of the first portion (1') or second portion (1"), respectively
   - the width of the first portion (1') and of the second portion (1") in the attachment region is significantly smaller than the width of the first (1') or second portion (1") in either end region.
65. Flexible web (1) according to clauses 56-62, wherein the web (1) has, in its unfolded state, a substantially rectangular shape.
66. Flexible web (1) according to clause 65 wherein the web (1) is folded along a folding line (L) situated such that the two resulting web portions (1', 1") are substantially equal.
67. Flexible web (1) according to clauses 58-66 wherein the web (1) area in the vicinity of the folding line (L) is made of a hydrophobic material while the remaining area of the web (1) is made of hydrophilic material.
68. Flexible web (1) according to clause 67 wherein the web (1) area made of a hydrophobic material is impregnated with an active or non-active substance.
69. Flexible web (1) according to clauses 67-68 wherein the hydrophobic material is softer than the hydrophilic material.
70. System for delivering, distributing and retaining a liquid, a gel or a cream substance into a vaginal or an anal cavity of a human or animal, comprising:
   - a web (1) according to any one of the clauses 46-69
   - an applicator (30) comprising at least one orifice at one end for delivering said substance
   **characterized in that**
   the end of the applicator (30) comprising said orifice can be releasably fixed into zone (Z) by means of the two extremities of the welding regions in the proximity of the folding line (L) of the web (1).
71. Kit for delivering a substance (60) into a vaginal or a rectal cavity (40,50) of a human or animal, comprising:
   - the web (1) according to any of clauses 1 to 37 or 46 to 69;
   - the applicator (30) according to any of clauses 38 to 43 or clause 70;
   - at least one substance (60) to be administered inside the vaginal or rectal cavity of a human or animal.
72. Kit according to clause 69 comprising a web (1) according to any of clauses 1 to 37, wherein the substance (60) is housed inside the pouch (10).
73. Kit according to clause 71 or 72 wherein the substance (60) is a liquid, a cream, a gel.
74. Kit according to clause 71 or 72 comprising a web (1) according to any of clauses 1 to 37, wherein the substance (60) is a tablet, capsule or suppository.
75. Kit according to any of clauses 71-73 comprising a web (1) according to any of clauses 1 to 37, wherein said substance (60) is placed inside the pouch (10) before the insertion of the web (1) in the vaginal or rectal cavity (40,50).
76. Kit according to any of clauses 71-73 wherein said substance (60) is placed inside the pouch (10) after the insertion of the web (10) in the vaginal or rectal cavity (40,50).
77. Use of a web (1) according to any of clauses 1-37 for delivery or administration of a substance (60) inside the vaginal or rectal cavity (40,50) of a human or animal.
78. Use of a web (1) according to any of clauses 1-37 or 46 to 69 for retaining bodily fluids.
79. Method of manufacturing the web (1) according to clauses 1- 37 comprising the steps of:
   a) applying the means of removal (4) on the material of the web (1) ;
   b)cutting the web (1) to the desired dimension;
   c) placing two sheets (16, 17) of the web (1) one above the other to form an overlapping area;
   d) fixing said two sheets (16, 17) together with stopping elements (11, 12, 13) for example by welding;
   e) optionally adding at least one blocking element (20) in the desired position.
80. Method of manufacturing the web (1) according to clause 79, comprising the steps of:
   a) delivering a roll of material to a conveyor;
   b) attaching the means of removal (4) on the web (1) such that areas of strong attachment alternate with areas of releasable attachment;
   c) cutting the roll of material together with the means of removal (4) at regular intervals to obtain rectangular shaped fabrics each having one area of strong attachment and one area of releasable attachment;
   d) folding each rectangular fabric of the preceding step by lifting it along a folding line that represents its middle width, thus obtaining two overlapping sheets (16, 17) of the web (1);
   e) welding together said two sheets of the web (1) along desired lines or areas defining a closed pouch (10) and next to it a blocking element (20).
81. Method of manufacturing the web (1) according to clause 80, comprising a further step before step d) to partially cut the roll of material along the line that represents the middle width of said rectangular fabric .

## Claims

1. System for delivering, distributing and retaining a liquid, a gel or a cream substance into a vaginal or an anal cavity of a human or animal, comprising an applicator (30) comprising at least one orifice at one end for delivering said substance
and a flexible web (1) provided with means of removal (4) from said vaginal or anal cavity, wherein
- a first portion (1') of the web (1) is folded over a remaining second portion (1") of the web (1) along a folding line (L),
- the first (1') and second (1") portions of the web (1) are maintained in a folded position by a plurality of welding regions (23), thereby :
i) defining a retaining pouch (2) having lateral edges corresponding to said welding regions (23) and a top opening and a bottom opening (O) for receiving the applicator (30), wherein the top is the side of the pouch closest to the folding line and the bottom is the side of the pouch furthest away from the folding line (L),
ii) also defining a zone (Z) which is delimited by the folding line (L) and by said top opening of the pouch and on the lateral sides by two further openings (O1, O2), situated at the extremities of the folding line (L),
wherein
the top opening is delimited by two extremities of the welding regions (23) and is configured to receive said applicator (30) and to keep said applicator (30) in a releasably fixed position when its end comprising the at least one orifice is in the zone (Z).

2. The system according to claim 1, wherein the welding zones (23) are welding lines symmetrically placed in respect to the longitudinal axis of the first portion (1') of the web (1).

3. The system according to claim 1 or 2, wherein the pouch (2) and/or zone (Z) is/are initially in a bloated state.

4. The system according to any of claims 1-3, wherein a third portion (1"') of the web (1) is folded over the second portion (1") of the web (1) along a second folding line (L') opposite to the first folding line (L), such that the first portion (1') and the third portion (1"') are identical in terms of area and means for maintaining the respective portions in folded position.

5. The system according to any preceding claim wherein the web (1) area in the vicinity of the folding line (L) is made of a hydrophobic material while the remaining area of the web (1) is made of hydrophilic material.

6. Kit for delivering a substance (60) into a vaginal or a rectal cavity (40,50) of a human or animal, comprising:
- the web (1) and applicator (30) according to any of claims 1 to 5; and
- at least one substance (60) to be administered inside the vaginal or rectal cavity of a human or animal.

7. Kit according to claim 6 wherein the substance (60) is a liquid, a cream or a gel.

8. Use of a web (1) according to any of claims 1-5 for delivery or administration of a substance (60) inside the vaginal or rectal cavity (40,50) of a human or animal.

9. Method of manufacturing the web (1) according to any of claims 1-5, comprising the steps of:
a) delivering a roll of material to a conveyor;
b) attaching the means of removal (4) on the web (1) such that areas of strong attachment alternate with areas of releasable attachment;
c) cutting the roll of material together with the means of removal (4) at regular intervals to obtain rectangular shaped fabrics each having one area of strong attachment and one area of releasable attachment;
d) folding each rectangular fabric of the preceding step by lifting it along a folding line that represents its middle width, thus obtaining two overlapping sheets (16, 17) of the web (1);
e) welding together said two sheets of the web (1) along desired lines or areas defining a closed pouch (10) and next to it a blocking element (20).

10. Method of manufacturing the web (1) according to claim 9, comprising a further step before step d) to partially cut the roll of material along the line that represents the middle width of said rectangular fabric .
